# EUROPEAN PATENT SPECIFICATION

(11) **EP 0 668 274 B1**
(45) Date of publication and mention of the grant of the patent: **26.08.1998**
(21) Application number: 95102353.0
(22) Date of filing: 20.02.1995
(51) Int. Cl.: C07D 321/06, A61K 31/18

(54) **Sulfonamidodioxepanes with hypoglycemic activity, methods of preparation, intermediates and salts thereof**
Sulfonamidodioxepane mit hypoglykämischen Eigenschaften, Methoden zu ihrer Herstellung, ihre Zwischenprodukte und Salze
Sulfonamidodioxépannes avec une activité hypoglycémique, méthodes pour leur préparation, leurs intermédiares et leurs sels

(30) Priority: 21.02.1994 HR 9401124
(43) Date of publication of application: 23.08.1995
(73) Proprietor: PLIVA, farmaceutska, kemijska, prehrambena i kozmeticka industrija, dionicko drustvo, 41000 Zagreb (HR)
(72) Inventor: Dumic, Miljenko, HR-41000 Zagreb (HR); Filic, Darko, HR-41000 Zagreb (HR); Vinkovic, Mladen, HR-42300 Cakovec (HR); Jamnicky, Blanka, HR-41000 Zagreb (HR)
(74) Representative: von Füner, Alexander, Dr.

(56) References cited:
- EP-A- 0 438 206
- TETRAHEDRON LETTERS., vol.34, no.22, 28 May 1993, OXFORD GB pages 3639 - 3642 MILJENKO DUMIC ET AL. '1-Sulfonyl-1a,2,6, 6a-tetrahydro-1H,4H-[1,3]-dioxepino[5,6-b] azirines: A Novel Class of Fused Dioxepines, Potent Hypoglycemic Agents'

## Description

The invention relates to novel sulfonamidodioxepanes, methods and intermediates for their preparation, the salts thereof, pharmaceutical preparations containing novel compounds and the use thereof.

Tetrahedron Letters 1993, pg.s 3639-3642 discloses derivatives of 1-sulfonyl-1a,2,6,6a-tetrahydro-1H,4H-[1,3]-dioxepino-[5,6-b]azirine, and suggests the use of these compounds as hypoglycemic agents.

Compounds of general formula I 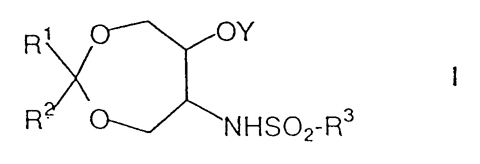 wherein
- R¹ and R²: represent a hydrogen atom, a straight-chain or branched alkyl with 1-4 C atoms or phenyl, or
- R¹ + R²: together represent an alkylidene group with 4-6 C atoms,
- R³: represents a straight-chain or branched alkyl with 1-4 C atoms, a straight-chain or branched mono- to perfluoroalkyl with 1-4 C atoms, and an o-, m- or p-substituted phenyl group 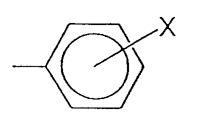 wherein
- X: represents a hydrogen atom, a lower straight-chain or branched alkyl with 1-4 C atoms, a trifluoromethyl group, a halogen atom with atomic number 9-53, a hydroxy, alkoxy, amino, alkyl- or dialkylamino, acylamino or hydroxyamino group, and
- Y: represents a hydrogen atom, a lower straight-chain or branched alkyl with 1-4 C atoms, a benzyl or Acyl group

-CO-R⁴

wherein
- R⁴: represents a lower straight-chain or branched all with 1-4 C atoms or a benzyl group,
and their physiologically acceptable salts have not been known so far.

Now it has been found that these compounds have valuable pharmacological properties, especially hypoglycemic activity, irrespective of their application route that can be intravenous, subcutaneous or oral. Hypoglycemic activity has been determined by standard tests on warm-blooded animals, e.g. mice.

In the compounds of the general formula I, alkyl and alkoxy represent methyl, ethyl, propyl, isopropyl, butyl, isobutyl, sec.-butyl and tert.-butyl, and methoxy, ethoxy, propoxy, isopropoxy, butoxy, sec.-butoxy, tert.-butoxy and isobutoxy groups. Acyl group is derived from aliphatic, arylaliphatic or aromatic carboxylic acids, e.g. formic acid, acetic acid, propionic acid, butyric acid, phenylacetic acid or benzoic acid.

With bases, the novel compounds of the general formula I form salts, which are also a subject of the present invention. Examples of such salts are alkali and alkaline-earth salts such as sodium, potassium, magnesium or calcium salts.

Novel compounds of the general formula I can be prepared according to the first process of the invention by condensing compounds of the general formula II 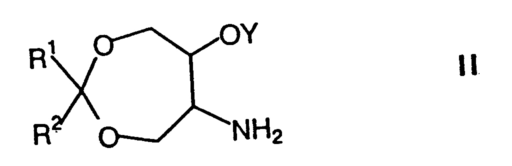 wherein
R¹, R² and Y have the above described meanings,
with reactive derivatives of sulfonic acids of the general formula III

R³SO₂-Z III

wherein
- R³: has the above described meanings and
- Z: represents a halogen atom with atomic number 9 to 17 or -OSO₂R³ group, wherein R³ has the above described meanings,
in water-miscible or water-unmiscible inert organic solvents, in the presence or absence of water, in the presence or absence of inorganic or organic acid-binding agents, at temperatures from -50°C to +50 °C preferably at -10°C to +10°C, and, optionally, converting the obtained compounds with inorganic bases or metal alcoholates into pharmaceutically acceptable salts.

Suitable inert solvents are e.g. hydrocarbons such as toluene or xylene, lower alcohols with up to 6 C atoms such as methanol or ethanol, ethers such as diethylether, dioxane or tetrahydrofuran, chlorinated hydrocarbons such as methylene chloride or chloroform, lower ketones with up to 6 C atoms such as acetone, methyl ethyl ketone or methyl isobutyl ketone, carboxylic acid esters such as ethyl acetate, carboxylic acid nitriles such as acetonitrile, amides such as dimethyl formamide or HMPT, sulfoxides such as dimethyl sulfoxide or sulfones such as sulfolane.

As suitable inorganic bases, alkali and alkaline-earth hydroxides, hydrogen carbonates, carbonates or phosphates, i.e. sodium or potassium and magnesium or calcium compounds can be used.

Suitable organic bases are tertiary amines such as triethylamine, dimethylaniline, pyridine, DBN or DBU.

Suitable starting materials of the general formula II are compounds which are appropriately substituted in accordance with the definition of symbols R¹, R² and Y as given at formula I. One such group of starting materials are 6-amino-1,3-dioxepane-5-ols, which can easily be prepared e.g. by hydrolysis of appropriate 6-acetylamino-5-chloro-1,3-dioxepanes (M. Sovak and R. Ranganathan, US 4,389,526) and amonolysis of epoxy-1,3-dioxepanes (M. Sovak and R. Ranganathan, EP 33 426; A.V. Rama Rao et al., Indian J. Chem**. 22B** (1983) 419).

According to the second process of the invention, the novel compounds of the general formula I can be prepared by reacting oxazolines of the general formula IV 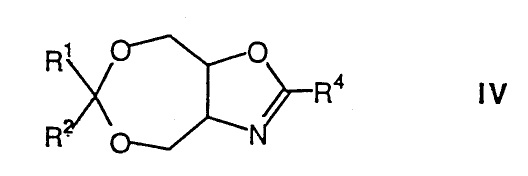 wherein
R¹, R² and R⁴ have the above described meanings,
   with reactive derivatives of sulfonic acids of general formula III,
   wherein
R³ and Z have the above described meanings,
in non-aqueous inert organic solvents, in the presence of inorganic or organic acid-binding agents and, optionally, hydrolyzing or alcoholyzing the obtained derivatives of the general formula I (Y = -COR⁴) into derivatives of formula I (Y = H) and, optionally, converting the obtained compounds with inorganic bases and metal alcoholates into pharmaceutically acceptable salts. The method of performing this process is identical to the method of the first process with the exception that it has to be performed in a non-aqueous medium.

Suitable starting materials of the general formula IV are compounds which are appropriately substituted in accordance with the definition of symbols R¹, R² and R⁴ as given at formula I. They can be easily prepared by dehydrohalogenation cyclisation of the corresponding 6-acylamino-5-chloro-1,3-dioxepanes (M. Dumić et al., Org. Prep. Proced. Int. **24,** (1992) 536 and ibid. **25,** (1992) 373).

According to the third process of the invention, novel compounds of the general formula I can be prepared by reacting epoxides of general formula V 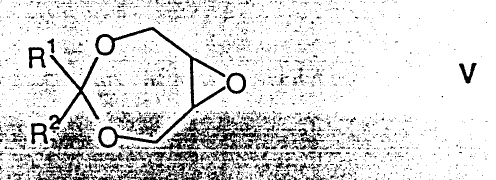 wherein
R¹ and R² have the above described meanings,
   with sulfonamides of general formula VI

   R³SO₂NH₂ VI

   wherein
R³ has the above described meanings,
by means of heating both reactants in the absence or in the presence of inert organic solvents such as e.g. aromates such as toluene or xylene, chlorinated hydrocarbons such as methylene chloride, chloroform or dichlorethane, carboxylic acid esters such as ethyl acetate, ethers such as diisopropylether or dioxane, amides such as dimethyl formamide, dimethyl acetamide or HMPT, sulfoxides such as dimethylsulfoxide, or sulfones such as sulfolane, at temperatures from 25°C to 300°C and preferably between 100 °C and 200 °C, and optionally, converting the obtained compounds with inorganic bases or metal alcoholates into pharmaceutically acceptable salts.

Suitable starting materials of the general formula V are compounds which are appropriately substituted in accordance with the definition of symbols R¹ and R² at formula I. They can be easily prepared by epoxydation of suitable dihydrodioxepines (J. Soulier et al., C.R. Acad. Sci. Ser. C. **280,** (1975) 681; W.J. Elliot et al., J. Org. Chem. **41,** (1976) 2469; A.J. Biloski, Synthesis **1980,** 810).

According to the fourth process of the invention, novel compounds of the general formula I can be prepared by hydrolyzing N-sulfonyl-dioxepinoazirines of the general formula VII 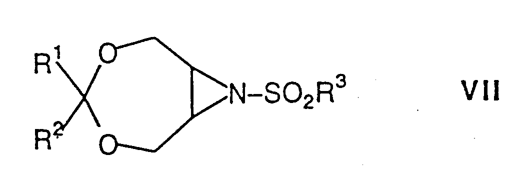 wherein
R¹, R² and R³ have the above described meanings,
in water or in water-miscible organic solvents, in the presence of inorganic bases, at temperatures from 0°C to 150°C, preferably at 50°C to 100°C, and, optionally, converting the obtained compounds with inorganic bases or metal alcoholates into pharmaceutically acceptable salts.

As organic solvents there can be used alcohols with up to 6 C atoms such as methanol or ethanol or tert.-butanol, ethers such as dioxane or tetrahydrofurane, chlorinated hydrocarbons such as methylene chloride or chloroform, ketones such as acetone, amides such as dimethylformamide or HMPT, amines such as pyridine, sulfoxides such as dimethylsulfoxid, or sulfones such as sulfolane. As bases, alkali metal hydroxides or carbonates, i.e. lithium, sodium or potassium compounds can be used.

Suitable starting materials of the general formula VII are compounds, which are appropriately substituted in accordance with the definition of symbols R¹, R² and R³ at formula I. They can be prepared by sulfonation of convenient dioxepinoazirines (M. Dumić et al., WO 93 04,067; Tetrahedron Lett. **34** (1993) 3639).

Optionally, novel compounds of the general formula I obtained according to the processes (1-4) of the invention, are being converted into their pharmaceutically acceptable salts by reacting the compounds of the general formula I with an equimolar amount of an inorganic base, alkali hydroxide e.g. sodium hydroxide, or alkali alcoholate such as sodium methylate, in inert organic solvents such as methanol, ethanol, acetone, toluene, diisopropylether.

Novel compounds of the general formula I prepared according to the processes of the invention or their pharmaceutically acceptable salts show a significantly to strongly expressed hypoglycemic activity in a model of diabetes in mice induced by streptozotocine, irrespective of the administration route that can be intravenous, subcutaneous or oral. For example, 4 hours after subcutaneous application of cis-6-sulfanylamido-1,3-dioxepane-5-ol in a dose of 20 mg/kg to mice with diabetes induced by streptozocine, a concentration of glucose in blood was reduced for 16.6%, i.e. the level of glucose was 83.4% of the level present in untreated hyperglycemic animals.

In view of the above, novel sulfonamidodioxepanes of the general formula I and their pharmaceutically acceptable salts represent effective hypoglycemic agents and by conventional processes of pharmaceutic technology, they can be converted into suitable pharmaceutical formulations such as tablets, pills, powders, capsules, granules, solutions, etc. of short-term or prolonged activity for therapy of *diabetes mellitus.*

The present invention is illustrated, yet in no way limited by the following examples.

### Example 1

A mixture of trans-6-amino-1,3-dioxepane-5-ol (0.30 g), 4-acetyl-aminobenzene-sulfochloride (0.58 g), pyridine (0.40 ml) and methylene chloride (10.0 ml) was stirred at the temperature of 0°C for 60 minutes. After evaporation of the solvent at reduced pressure, the evaporation residue was chromatographed on a silica gel column by elution with ethyl acetate.

Trans-6-(4-acetylaminobenzenesulfonamido)-1,3-dioxepane-5-ol was obtained. M.p. 210-211°C / ethyl acetate-methanol (9.5:0.5)

### Example 2

A mixture of cis-2-methyl-3a,4,8,8a-tetrahydro-6H-[1,3]-dioxepino-[5,6-d]oxazole (1.00 g), 4-acetylaminobenzenesulfochloride (1.50 g), pyridine (1.05 ml) and methylene chloride (60.0 ml) was stirred at room temperature for 90 minutes. After addition of water (10.0 ml), the mixture was stirred for further 15 minutes at the same temperature. The product was extracted with methylene chloride, the extract was dried over anhydrous sodium sulfate, methylene chloride was evaporated at reduced pressure and the evaporation residue was chromatographed on a silica gel column by elution with a mixture of ethyl acetate/methanol (9.8:0.2).

Cis-6-(4-acetylaminobenzenesulfonamido)-5-acetoxy-1,3-dioxepane was obtained. M.p. 184-186 °C / ethyl acetate-methanol (6:1).

A mixture of cis-6-(4-acetylaminobenzenesulfonamido)-1,3-dioxepane (0.150 g), 25% ammonia (3.0 ml) and 96% ethanol (6.0 ml) was stirred at room temperature for 3 hours. The mixture was then evaporated to dryness at reduced pressure and by the recrystallization of the evaporation residue from ethyl acetate/methanol (1:1) mixture, cis-6-(4-acetylaminobenzenesulfonamido)-1,3-dioxepane-5-ol was obtained, m.p. 161-163 °C.

### Example 3

A mixture of 5,6-epoxy-1,3-dioxepane (0.5g) and of 4-acetylaminobenzene-sulfonamide (0.92 g) was heated in a sealed ampule at the temperature of 150 °C for 15 minutes. The mixture was cooled to room temperature, chromatographed on a silica gel column by elution with a mixture of ethyl acetate-methanol (9.5:0.5) and trans-6-(4-acetylaminobenzenesulfonamido)-1,3-dioxepane-5-ol was obtained. M.p. 208-210 °C / ethyl acetate-methanol (6:1).

### Example 4

A mixture of 1-(4-acetylaminobenzenesulfonyl)-4,4-dimethyl-1a,2,6,6a-tetrahydro-1H,4H-[1,3]-dioxepino[5,6-b]azirine (0.33 g), potassium hydroxide (0.14 g) and water (2.6 ml) was boiled with reflux for 60 minutes. The mixture was cooled to room temperature, acidified with diluted hydrochloric acid to pH 6.5 and evaporated to dryness at reduced pressure. The chromatography of the evaporation residue on a silica gel column by elution with a mixture of ethyl acetate/methanol (9.8:0.2) yielded trans-6-(4-acetylaminobenzene-sulfonamido)-2.2-dimethyl-1,3-dioxepane-5-ol, m.p. 203-205 °C / methylene chloride- methanol (9:1), and trans-6-sulfanylamido-2,2-dimethyl-1,3-dioxepane-5-ol.

### Example 5

A mixture of 1-(4-acetylaminobenzenesulfonyl)-1a,2,6,6a-tetrahydro-1H,4H-[1,3{-dioxepino-[5,6-b]azirine (0.30 g), potassium hydroxide (0.10 g) and water (2.5 ml) was boiled with reflux for 60 minutes. The mixture was cooled to room temperature, acidified with diluted hydrochloric acid to pH 6.5 and evaporated to dryness at reduced pressure. The evaporation residue was chromatographed on a silica gel column by elution with a mixture of methylene chloride-methanol (10:1).

Trans-6-(4-acetylaminobenzenesulfonamido)-1,3-dioxepane-5-ol, m.p. 209-211 °C / ethyl acetate-methanol (1:1), and trans-6-sulfanylamido-1,3-dioxepane-5-ol, m.p. 162-164 °C / ethyl acetate-methanol (1:1), were obtained.

### Example 6

To a mixture of cis-6-amino-1,3-dioxepane-5-ol (0.30 g) and pyridine (0.40 ml) in methylene chloride (10.0 ml), a solution of 4-acetylaminobenzenesulfochloride (0.58 g) in methylene chloride (35.0 ml) was added drop by drop within 90 minutes at the temperature of 0 °C. Subsequently, the mixture was stirred at the same temperature for further 15 minutes and then evaporated to dryness at reduced pressure. Chromatography of the evaporation residue on a silica gel column by elution with a mixture of methylene chloride-methanol (8:2) yielded cis-6-(4-acetylaminobenzene-sulfonamido)-1,3-dioxepane-5-ol, m.p. 159-161 °C / ethyl acetate-methanol (2:1).

## Claims

1. Compounds of general formula I 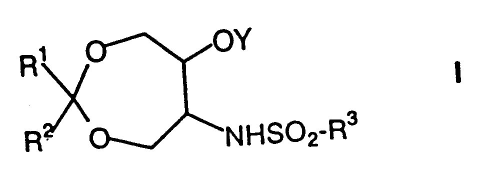 wherein
R¹ and R² represent a hydrogen atom, a straight-chain or branched alkyl with 1-4 C atoms or phenyl, or
R¹ + R² together represent alkylidene group with 4-6 C atoms,
R³ represents a straight-chain or branched alkyl with 1-4 C atoms, a straight-chain or branched mono- to perfluoroalkyl with 1-4 C atoms, and an o-, m- or p-substituted phenyl group 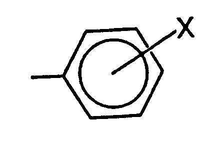 wherein
X represents a hydrogen atom, a lower straight-chain or branched alkyl with 1-4 C atoms, trifluoromethyl group, a halogen atom with atomic number 9-53, hydroxy, alkoxy, amino, alkyl- or dialkylamino, acylamino or hydroxyamino group, and
Y represents a hydrogen atom, a lower straight-chain or branched alkyl with 1-4 C atoms, benzyl or Acyl group
-CO-R⁴
wherein
R⁴ represents a lower straight-chain or branched all with 1-4 C atoms, or benzyl group,
and their physiologically acceptable salts.

2. Compound according to claim 1, characterized in that R¹ = R² = H, cis, Y = H, R³ = 4-CH₃CONH-C₆H₄-, and its pharmaceutically acceptable salts.

3. Compound according to claim 1, characterized in that R¹ = R² = H, trans, Y = H, R³ = 4-CH₃CONH-C₆H₄-, and its pharmaceutically acceptable salts.

4. Compound according to claim 1, characterized in that R¹ = R² = H, cis, Y = H, R³ = 4-H₂N-C₆H₄-, and its pharmaceutically acceptable salts.

5. Compound according to claim 1, characterized in that R¹ = R² = H, trans, Y = H, R³ = 4-H₂N-C₆H₄-, and its pharmaceutically acceptable salts.

6. Compound according to claim 1, characterized in that R¹ = R² = H, cis, Y = H, R³ = C₆H₅-, and its pharmaceutically acceptable salts.

7. Compound according to claim 1, characterized in that R¹ = R² = H, trans, Y = H, R³ = C₆H₅-, and its pharmaceutically acceptable salts.

8. Compound according to claim 1, characterized in that R¹ = R² = H, cis, Y = H, R³ = CH₃-, and its pharmaceutically acceptable salts.

9. Compound according to claim 1, characterized in that R¹ = R² = H, trans, Y = H, R³ = CH₃-, and its pharmaceutically acceptable salts.

10. Compound according to claim 1, characterized in that R¹ = R² = CH₃-, cis, Y = H, R³ = 4-H₂N-C₆H₄-, and its pharmaceutically acceptable salts.

11. Compound according to claim 1, characterized in that R¹ = R² = CH₃-, trans, Y = H, R³ = 4-H₂N-C₆H₄-, and its pharmaceutically acceptable salts.

12. Compound according to claim 1, characterized in that R¹ = R² = CH₃-, cis, Y = H, R³ = 4-CH₃CONH-C₆H₄-, and its pharmaceutically acceptable salts.

13. Compound according to claim 1, characterized in that R¹ = R² = CH₃-, trans, Y = H, R³ = 4-CH₃CONH-C₆H₄-, and its pharmaceutically acceptable salts.

14. Process for the preparation of novel compounds of the general formula I and their pharmaceutically acceptable salts according to claim 1, characterized in that compounds of general formula II 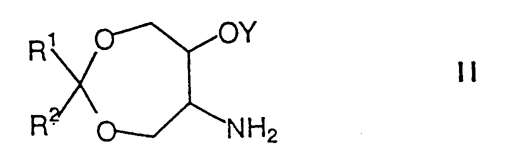 wherein
R¹, R² and Y have the meanings described in claim 1,
are reacted with reactive derivatives of sulfonic acids of general formula III
R³SO₂-Z III
wherein
R³ has the meanings described in claim 1, and
Z represents a halogen atom with atomic number 9 to 17 or -OSO₂R³ group,
wherein
R³ has the meanings described in claim 1,
in water-miscible or water-unmiscible inert organic solvents, in the presence or absence of water, in the presence or absence of inorganic or organic acid-binding agents, at a temperature from -50°C to +50°C, and, optionally, the obtained compounds are converted with inorganic bases or metal alcoholates into pharmaceutically acceptable salts.

15. Process for the preparation of novel compounds of the general formula I and their pharmaceutically acceptable salts according to claim 1, characterized in that compounds of general formula IV 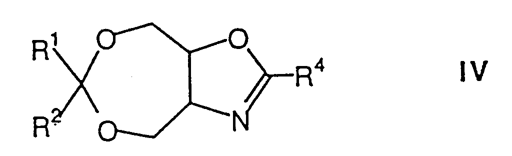 wherein
R¹, R² and R⁴ have the meanings as described in claim 1,
are reacted with reactive derivatives of sulfonic acids of general formula III,
R³SO₂-Z III
wherein
R³ and Z have the meanings as described in claim 14,
in non-aqueous inert organic solvents, in the presence of inorganic or organic acid-binding agents and, optionally, the obtained derivatives of the general formula I (Y = -COR⁴) are hydrolyzed or alcoholyzed into derivatives of formula I (Y = H) and, if desired, the obtained compounds are converted with inorganic bases or metal alcoholates into pharmaceutically acceptable salts.

16. Process for the preparation of novel compounds of general formula I and of their pharmaceutically acceptable salts according to claim 1, characterized in that compounds of general formula V 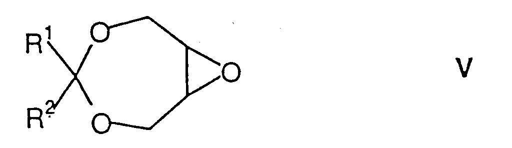 wherein
R¹ and R² have the meanings described in claim 1,
are reacted with sulfonamides of general formula VI
R³SO₂NH₂ VI
wherein
R³ has the meanings described in claim 1,
in the absence or presence of inert organic solvents, at temperatures from 25°C to 300°C and, optionally, the obtained compounds are converted with inorganic bases or metal alcoholates into pharmaceutically acceptable salts.

17. Process for the preparation of novel compounds of the general formula I and of their pharmaceutically acceptable salts according to claim 1, characterized in that compounds of general formula VII 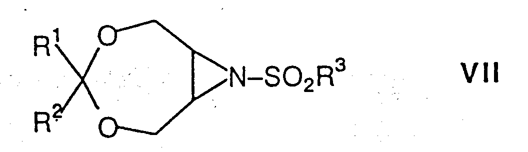 wherein
R¹, R² and R³ have the meanings described in claim 1,
are hydrolyzed in water or in water-miscible organic solvents, in the presence of inorganic bases, at temperatures from 0 °C to 150 °C and, optionally, the obtained compounds are converted with inorganic bases or metal alcoholates into pharmaceutically acceptable salts.

18. Use of compounds according to claims 1-13 as intermediates for the synthesis of hypoglycemic agents.

19. Pharmaceutical preparations for the therapy of *diabetes mellitus*, characterized in that they comprise a sulfonamide of the general formula I according to claims 1-13 as the active component.

## Patentansprüche

1. Verbindungen der allgemeinen Formel I 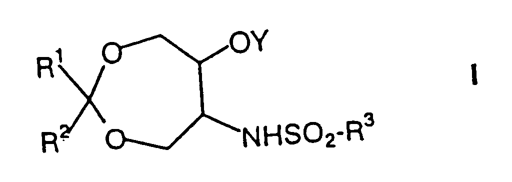 worin
R¹ und R² ein Wasserstoffatom, ein geradekettiges oder verzweigtes Alkyl mit 1-4 C Atomen oder Phenyl darstellen, oder
R¹ + R² zusammen eine Alkylidengruppe mit 4-6 C Atomen darstellen,
R³ ein geradekettiges oder verzweigtes Alkyl mit 1-4 C Atomen, ein geradekettiges oder verzweigtes Mono- bis Perfluoroalklyl mit 1-4 C Atomen, und eine o-, m- oder p-substituierte Phenylgruppe 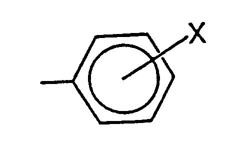 darstellt, worin
X ein Wasserstoffatom, ein niedriges geradekettiges oder verzweigtes Alkyl mit 1-4 C Atomen, Trifluoromethylgruppe, ein Halogenatom mit Ordnungszahl 9-53, Hydroxy, Alkoxy, Amino, Alkyl- oder Dialkylamino, Acylamino oder Hydroxyaminogruppe darstellt, und
Y ein Wasserstoffatom, ein niedriges geradekettiges oder verzweigtes Alkyl mit 1-4 C Atomen, Benzyl- oder Acylgruppe
-CO-R⁴
darstellt, worin
R⁴ ein niedriges geradekettiges oder verzweigtes Alkyl mit 1-4 C Atomen oder Benzylgruppe darstellt,
und ihre physiologisch annehmbaren Salze.

2. Verbindung gemäß Anspruch 1, dadurch gekennzeichnet, daß R¹ = R² = H, cis, Y = H, R³ = 4-CH₃CONH-C₆H₄-, und ihre pharmazeutisch annehmbaren Salze.

3. Verbindung gemäß Anspruch 1, dadurch gekennzeichnet, daß R¹ = R² = H, trans, Y = H, R³ = 4-CH₃CONH-C₆H₄-, und ihre pharmazeutisch annehmbaren Salze.

4. Verbindung gemäß Anspruch 1, dadurch gekennzeichnet, daß R¹ = R² = H, cis, Y = H, R³ = 4-H₂N-C₆H₄-, und ihre pharmazeutisch annehmbaren Salze.

5. Verbindung gemaß Anspruch 1 , dadurch gekennzeichnet, daß R¹ = R² = H, trans, Y = H, R³ = 4-H₂N-C₆H₄-, und ihre pharmazeutisch annehmbaren Salze.

6. Verbindung gemäß Anspruch 1, dadurch gekennzeichnet, daß R¹ = R² = H, cis, Y = H, R³ = -C₆H₅-, und ihre pharmazeutisch annehmbaren Salze.

7. Verbindung gemaß Anspruch 1, dadurch gekennzeichnet, daß R¹ = R² = H, trans, Y = H, R³ = -C₆H₅-, und ihre pharmazeutisch annehmbaren Salze.

8. Verbindung gemaß Anspruch 1, dadurch gekennzeichnet, daß R¹ = R² = H, cis, Y = H, R³ = CH₃-, und ihre pharmazeutisch annehmbaren Salze.

9. Verbindung gemäß Anspruch 1, dadurch gekennzeichnet, daß R¹ = R² = H, trans, Y = H, R³ = CH₃-, und ihre pharmazeutisch annehmbaren Salze.

10. Verbindung gemaß Anspruch 1, dadurch gekennzeichnet, daß R¹ = R² = CH₃-, cis, Y = H, R³ = 4-H₂N-C₆H₄-, und ihre pharmazeutisch annehmbaren Salze.

11. Verbindung gemäß Anspruch 1, dadurch gekennzeichnet, daß R¹ = R² = CH₃-, trans, Y = H, R³ = 4-H₂N-C₆H₄-, und ihre pharmazeutisch annehmbaren Salze.

12. Verbindung gemäß Anspruch 1, dadurch gekennzeichnet, daß R¹ = R² = CH₃-, cis, Y = H, R³ = 4-CH₃CONH-C₆H₄-, und ihre pharmazeutisch annehmbaren Salze.

13. Verbindung gemäß Anspruch 1, dadurch gekennzeichnet, daß R¹ = R² = CH₃-, trans, Y = H, R³ = 4-CH₃CONH-C₆H₄-, und ihre pharmazeutisch annehmbaren Salze.

14. Verfahren zur Herstellung von neuen Verbindungen der allgemeinen Formel I und ihren pharmazeutisch annehmbaren Salzen gemäß Anspruch 1, dadurch gekennzeichnet, daß die Verbindungen der allgemeinen Formel II 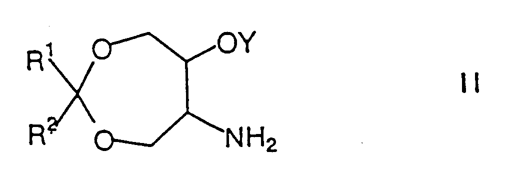 worin
R¹, R² und Y die im Anspruch 1 beschriebenen Bedeutungen haben,
mit reaktiven Derivaten von Sulfonsäuren allgemeiner Formel III
R³SO₂-Z III
worin
R³ die im Anspruch 1 beschriebenen Bedeutungen hat,
Z ein Halogenatom mit Ordnungszahl 9 bis 17 oder -OSO₂R³-Gruppe darstellt, worin
R³ die im Anspruch 1 beschriebenen Bedeutungen hat,
in mit Wasser mischbaren oder mit Wasser unmischbaren inerten organischen Lösungsmitteln, in An- oder Abwesenheit von Wasser, in An- oder Abwesenheit von anorganischen oder organischen säurebindenden Mitteln, bei einer Temperatur von -50°C bis +50°C umgesetzt werden und gegebenenfalls die erhaltenen Verbindungen mit anorganischen Basen oder Metallalkoholaten in pharmazeutisch annehmbare Salze überführt werden.

15. Verfahren zur Herstellung von neuen Verbindungen der allgemeinen Formel I und ihren pharmazeutisch annehmbaren Salzen gemäß Anspruch 1, dadurch gekennzeichnet, daß die Verbindungen der allgemeinen Formel IV 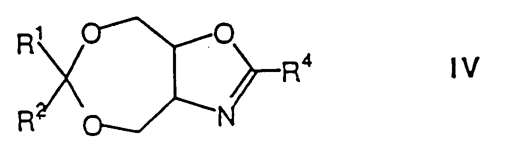 worin
R¹, R² und R⁴ die im Anspruch 1 beschriebenen Bedeutungen haben,
mit reaktiven Derivaten von Sulfonsäuren der allgemeinen Formel III,
R³SO₂-Z III
worin
R³ und Z die im Anspruch 14 beschriebenen Bedeutungen haben,
in nichtwässerigen inerten Lösungmitteln, in Anwesenheit von anorganischen oder organischen säurebindenden Mitteln umgesetzt werden, und gegebenenfalls die erhaltenen Derivate der allgemeinen Formel I (Y = -COR⁴) zu Derivaten der Formel I (Y = H) hydrolysiert oder alkoholysiert werden und, falls erwünscht, die erhaltenen Verbindungen mit anorganischen Basen oder Metallalkoholaten in pharmazeutisch annehmbare Salze überführt werden.

16. Verfahren zur Herstellung von neuen Verbindungen der allgemeninen Formel I und ihren pharmazeutisch annehmbaren Salzen gemäß Anspruch 1, dadurch gekennzeichnet, daß die Verbindungen der allgemeinen Formel V 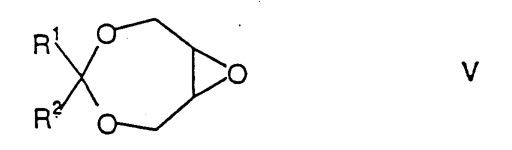 worin R¹ und R² die im Anspruch 1 beschriebenen Bedeutungen haben, mit Sulfonamiden der allgemeinen Formel VI
R³SO₂NH₂ VI
worin
R³ die im Anspruch 1 beschriebenen Bedeutungen hat,
in An- oder Abwesenheit von inerten organischen Lösungsmitteln, bei Temperaturen von 25°C bis 300°C umgesetzt werden, und gegebenenfalls die erhaltenen Verbindungen mit anorganischen Basen oder Metallalkoholaten in pharmazeutisch annehmbare Salze uberführt werden.

17. Verfahren zur Herstellung von neuen Verbindungen der allgemeinen Formel I und ihren pharmazeutisch annehmbaren Salzen gemäß Anspruch 1, dadurch gekennzeichnet, daß die Verbindungen der allgemeinen Formel VII 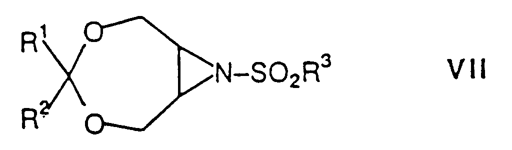 worin
R¹, R² und R³ die im Anspruch 1 beschriebenen Bedeutungen haben,
in Wasser oder in mit Wasser mischbaren organischen Lösungsmitteln, in Anwesenheit von anorganischen Basen, bei Temperaturen von 0°C bis 150°C hydrolysiert werden, und gegebenenfalls die erhaltenen Verbindungen mit anorganischen Basen oder Metallalkoholaten in pharmazeutisch annehmbare Salze überführt werden.

18. Verwendung von Verbindungen gemäß Ansprüchen 1-13 als Zwischenverbindungen zur Synthese von hypoglykämischen Mitteln.

19. Pharmazeutische Zubereitungen zur Behandlung von *Diabetes mellitus*, dadurch gekennzeichnet, daß sie ein Sulfonamid der allgemeinen Formel I gemäß Ansprüchen 1-13 als Wirkstoff enthalten.

## Revendications

1. Composés de la formule générale I 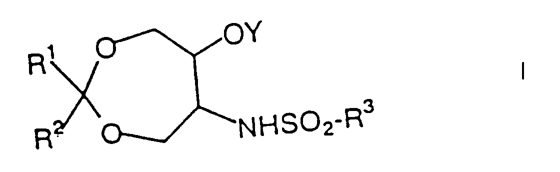 dans laquelle
R¹ et R² représentent chacun un atome d'hydrogène un radical alkyle possédant de 1 à 4 atomes de carbone, à chaîne droite ou à chaîne ramifiée, ou un radical phényle, ou bien
R¹ + R² représentent ensemble un radical alkylidène possédant de 4 à 6 atomes de carbone,
R³ représente un radical alkyle comportant de 1 à 4 atomes de carbone, à chaîne droite ou à chaîne ramifiée, un radical monoperfluoralkyle ou perfluoralkyle, comportant de 1 à 4 atomes de carbone, à chaîne droite ou à chaîne ramifiée, ou un radical phényle o-, m- ou p-substitué 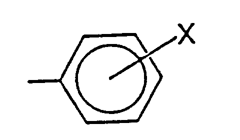 où
X représente un atome d'hydrogène, un radical alkyle inférieur comportant de 1 à 4 atomes de carbone, à chaîne droite ou à chaîne ramifiée, un radical trifluorométhyle, un atome d'halogéne possédant un nombre atomique de 9 à 53, un radical hydroxyle, alcoxy, amino, alkyl- ou dialkylamino, acylamino ou hydroxylamino et
Y représente un atome d'hydrogène, un radical alkyle inférieur comportant de 1 à 4 atomes de carbone, à chaîne linéaire ou à chaîne ramifiée, un radical benzyle ou acyle
-CO-R⁴
où
R⁴ représente un radical alkyle inférieur comportant de 1 à 4 atomes de carbone, à chaîne droite ou à chaîne ramifiée, ou un radical benzyle,
ainsi que leurs sels physiologiquement acceptables.

2. Composé suivant la revendication 1, caractérisé en ce que R¹ = R² = H, cis, Y=H, R³ = 4-CH₃CONH-C₆C₄- et ses sels pharmaceutiquement acceptables.

3. Composé suivant la revendication 1, caractérisé en ce que R¹ = R² = H, trans, Y=H, R³ = 4-CH₃CONH-C₆H₄- et ses sels pharmaceutiquement acceptables.

4. Composé suivant la revendication 1, caractérisé en ce que R¹ = R² = H, cis, Y=H, R³ = 4-H₂N-C₆H₄-, et ses sels pharmaceutiquement acceptables.

5. Composé suivant la revendication 1, caractérisé en ce que R¹ = R² = H, trans, Y=H, R³ = 4-H₂N-C₆H₄-, et ses sels pharmaceutiquement acceptables.

6. Composé suivant la revendication 1, caractérisé en ce que R¹ = R² = H, cis, Y=H, R³ = C₆C₅- et ses sels pharmaceutiquement acceptables.

7. Composé suivant la revendication 1, caractérisé en ce que R¹ = R² = H, trans, Y=H, R³ = C₆H₅- et ses sels pharmaceutiquement acceptables.

8. Composé suivant la revendication 1, caractérisé en ce que R¹ = R² = H, cis, Y=H, R³ = CH₃- et ses sels pharmaceutiquement acceptables.

9. Composé suivant la revendication 1, caractérisé en ce que R¹ = R² = H, trans, Y=H, R³ = CH₃- et ses sels pharmaceutiquement acceptables.

10. Composé suivant la revendication 1, caractérisé en ce que R¹ = R² = CH₃-, cis, Y=H, R³ = 4-H₂N-C₆C₄- et ses sels pharmaceutiquement acceptables.

11. Composé suivant la revendication 1, caractérisé en ce que R¹ = R² = CH₃-, trans, Y=H, R³ = 4-H₂N-C₆C₄- et ses sels pharmaceutiquement acceptables.

12. Composé suivant la revendication 1, caractérisé en ce que R¹ = R² = CH₃-, cis, Y=H, R³ = 4-CH₃CONH-C₆C₄- et ses sels pharmaceutiquement acceptables.

13. Composé suivant la revendication 1, caractérisé en ce que R¹ = R² = CH₃, trans, Y=H, R³ = 4-CH₃CONH-C₆C₄- et ses sels pharmaceutiquement acceptables.

14. Procédé de préparation de nouveaux composés de la formule générale I et de leurs sels pharmaceutiquement acceptables, suivant la revendication 1, caractérisé en ce que l'on fait réagir des composés de la formule générale II 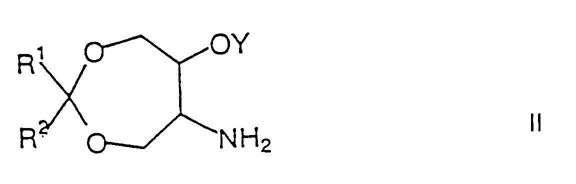 dans laquelle
R¹, R² et Y possèdent les significations qui leur ont été attribuées dans la revendication 1
avec des dérivés réactifs d'acides sulfoniques de la formule générale III
R³SO₂-Z III
dans laquelle
R³ possède les significations qui lui ont été attribuées dans la revendication 1 et
Z représente un atome d'halogène avec un nombre atomique de 9 à 17 ou un radical -OSO₂R³,
où
R³ possède les significations qui lui ont été attribuées dans la revendication 1,
dans des solvants organiques inertes miscibles à l'eau ou non miscibles à l'eau, en présence ou en l'absence d'eau, en présence ou en l'absence d'agents tant les acides, inorganiques ou organiques, à une température qui varie de -50°C à +50°C, et on convertit éventuellement les composés obtenus avec des bases inorganiques ou des alcoolates de métaux en sels pharmaceutiquement acceptables.

15. Procédé de préparation de nouveaux composés de la formule générale I et de leurs sels pharmaceutiquement acceptables suivant la revendication 1, caractérisé en ce que l'on fait réagir des composés de la formule générale IV 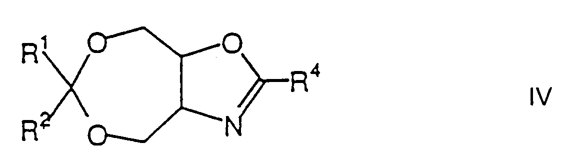 dans laquelle
R¹, R² et R⁴ possèdent les significations qui leur ont été attribuées dans la revendication 1,
avec des dérivés réactifs d'acides sulfoniques de la formule générale III
R³SO₂-Z III
dans laquelle
R³ et Z possèdent les significations qui leur ont été attribuées dans la revendication 14,
dans des solvants organiques inertes non aqueux, en présence d'agents liant les acides, inorganiques ou organiques et on hydrolyse ou alcoolyse éventuellement les dérivés obtenus de la formule générale I (Y = -COR⁴) en dérivés de la formule I (Y = H) et, si on le souhaite, on convertit les composés obtenus avec des bases inorganiques ou des alcoolates de métaux en sels pharmaceutiquement acceptables.

16. Procédé de préparation de nouveaux composés de la formule générale I et de leurs sels pharmaceutiquement acceptables, suivant la revendication 1, caractérisé en ce que l'on fait réagir des composés de la formule V 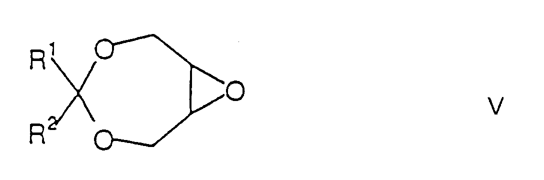 dans laquelle
R¹ et R² possèdent les significations qui leur ont été attribuées dans la revendication 1,
avec des sulfonamides de la formule générale VI
R³SO₂NH₂ VI
dans laquelle
R³ possède les significations qui lui ont été attribuées dans la revendication 1,
en l'absence ou la présence de solvants organiques inertes, à des températures qui varient de 25°C à 300°C et on convertit éventuellement les composés obtenus avec des bases inorganiques ou des alcoolates de métaux en sels pharmaceutiquement acceptables.

17. Procédé de préparation des nouveaux composés de la formule générale I et de leurs sels pharmaceutiquement acceptables suivant la revendication 1, caractérisé en ce que l'on hydrolyse des composés de la formule générale VII 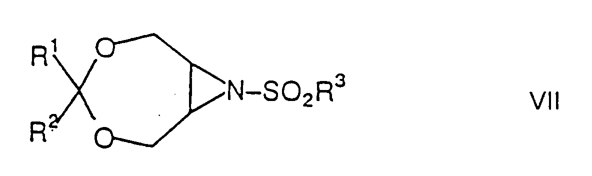 dans laquelle
R¹, R² et R³ possèdent les significations qui leur ont été attribuées dans la revendication 1,
dans de l'eau ou dans des solvants miscibles à l'eau, en présence de bases inorganiques, à des températures de 0°C à 150°C et on convertit éventuellement les composés obtenus avec des bases inorganiques ou des alcoolates de métaux en sels pharmaceutiquement acceptables.

18. Utilisation de composés suivant l'une quelconque des revendications 1 à 13, à titre d'intermédiaires pour la synthèse d'agents hypoglycémiques.

19. Préparations pharmaceutiques destinées au traitement thérapeutique du *diabetes mellitus* ou diabète sucré, caractérisé en ce qu'elles comprennent un sulfonamide de la formule générale I suivant l'une quelconque des revendications 1 à 13, à titre de composant actif.
